# EUROPEAN PATENT APPLICATION

(11) **EP 2 535 422 A2**
(43) Date of publication of application: **19.12.2012**
(21) Application number: 12183830.4
(22) Date of filing: 10.06.2008
(51) Int. Cl.: C12Q 1/02, C12Q 1/04, C12Q 1/34

(54) **Selection of useful fungal strains**

(30) Priority: 15.06.2007 US 934677 P
(62) Divisional of application: 08768332.2
(71) Applicant: Danisco US Inc., Palo Alto, CA 94304 (US)
(72) Inventor: Bodie, Elizabeth, Palo Alto, CA 94304 (US); Larenas, Edmund, Palo Alto, CA 94304 (US)
(74) Representative: Andrews, Robert

(57) **Abstract**

The present invention provides methods for screening for a desired filamentous fungus that produces cellulase more efficiently and/or with increased specific activity. It also provides filamentous fungi that produce cellulase more efficiently.

## Description

### 1. CROSS-REFERENCES TO RELATED APPLICATION

The present application claims benefit of and priority to U.S. Provisional Application Ser. No. US 60/934,677, filed June 15, 2007, which is incorporated herein by reference in its entirety.

### 2. GOVERNMENT SUPPORT

Portions of this work were funded by Subcontract No. ZCO-30017-01 with the National Renewable Energy Laboratory under Prime Contract No. DE-AC36-99GO10337 with the U.S. Department of Energy. Accordingly, the United States Government may have certain rights in this invention.

### 3. INTRODUCTION

Filamentous fungi are efficient producers of cellulase enzymes and have been exploited for their ability to produce these enzymes. Cellulases are valuable commercially in the textile, detergent and paper industries, and increasingly for the production of biofuels, which requires the hydrolysis of plant matter to fermentable sugars. Three major types of enzymatic activities are found: exoglucanases or exocellobiohydrolases, endoglucanses and β-glucosidases. These three different types of cellulase enzymes act synergistically to convert cellulose to glucose. There is a need to identify desired filamentous fungi, especially filamentous fungi with desired protein yield and production, e.g., high yield and/or production of cellulases enzymes.

### 4. SUMMARY

The present teachings are based, at least in part, on the discovery that certain parameters can be combined in the process of screening for desired filamentous fungi. Accordingly the present teachings provide methods for screening for a desired filamentous fungus based on at least two parameters, *e.g.*, parameters associated with metabolism and carbon usage, and filamentous fungi obtained thereof.

In some embodiments, the present teachings provide a method for screening for a desired filamentous fungus. Here a population of testing filamentous fungi is screened for a subpopulation of filamentous fungi with a predetermined rate of metabolism. The desired filamentous fungus is selected from the subpopulation of filamentous fungi based on a pre-determined selection parameter that indicates carbon usage efficiency of the testing filamentous fungus. In some embodiments, the invention provides a filamentous fungus obtained by this screening method.

In some embodiments, the present teachings provide a method for screening for a desired filamentous fungus. Here a population of testing filamentous fungi is screened based on a first selection parameter and a second selection parameter. The first selection parameter indicates the rate of metabolism of a testing filamentous fungus and the second selection parameter indicates the ability of a testing filamentous fungus to resist an antibiotic.

In some embodiments, the present teachings provide a method for screening for a desired filamentous fungus. Here a population of testing filamentous fungi is pre-selected against an antibiotic and is screened based on a pre-determined selection parameter that indicates the rate of metabolism of a testing filamentous fungus.

These and other features of the present teachings are set forth below.

### 5. BRIEF DESCRIPTION OF THE DRAWINGS

The skilled artisan will understand that the drawings are for illustration purposes only. The drawings are not intended to limit the scope of the present teachings in anyway.

Figure 1 shows the improvement protein production in A83 strain as compared to the 41 G strain.

Figure 2 shows the specific activity advantage of the 41 G strain as compared to the A83 strain.

Figure 3 shows the evolution of the A83 strain and the improvements in yield (grey box) and productivity (white box).

Figure 4 shows the performance of 41G and A83 in a saccharification assay.

### 6. DETAILED DESCRIPTION OF VARIOUS EMBODIMENTS

The present teachings will now be described in detail by way of reference only using the following definitions and examples. Unless defined otherwise herein, all technical and scientific terms used herein have the same meaning as commonly understood by one of ordinary skill in the art to which this invention belongs. Numeric ranges are inclusive of the numbers defining the range. The headings provided herein are not limitations of the various aspects or embodiments which can be had by reference to the specification as a whole. Accordingly, the terms defined immediately below are more fully defined by reference to the specification as a whole.

The term "polypeptide" as used herein refers to a compound made up of a single chain of amino acid residues linked by peptide bonds. The term "protein" as used herein is used interchangeably with the term "polypeptide."

The term "nucleic acid" and "polynucleotide" are used interchangeably and encompass DNA, RNA, cDNA, single stranded or double stranded and chemical modifications thereof. Because the genetic code is degenerate, more than one codon may be used to encode a particular amino acid, and the present invention encompasses all polynucleotides, which encode a particular amino acid sequence.

The terms "recovered", "isolated", and "separated" are used interchangeably herein to refer to a protein, cell, nucleic acid, amino acid etc. that is removed from at least one component with which it is naturally associated.

As used herein, the term "gene" refers to a polynucleotide (e.g., a DNA segment) involved in producing a polypeptide chain, that may or may not include regions preceding and following the coding region, e.g. 5' untranslated (5' UTR) or "leader" sequences and 3' UTR or "trailer" sequences, as well as intervening sequences (introns) between individual coding segments (exons).

As used herein, the term "expression" refers to the process by which a polypeptide is produced based on the nucleic acid sequence of a gene. The process includes both transcription and translation.

As used herein, the term "expression" refers to the process by which a polypeptide is produced based on the nucleic acid sequence of a gene. The process includes both transcription and translation.

The term "secreted protein" refers to a region of a polypeptide that is released from a cell during protein secretion. In some embodiments, the secreted protein is the protein that is released or cleaved from a recombinant fusion polypeptide of the invention.

The term "secretion" refers to the selective movement of a protein across a membrane in a host cell to the extracellular space and surrounding media

As used herein, the terms "produced" or "production of," especially as related to proteins or enzymes, refers to the expression and/or secretion of the protein or enzyme.

As used herein, the term "culturing" refers to growing a population of cells under suitable conditions in a liquid, semi-solid or solid medium.

As used herein, "substituted" and "modified" are used interchangeably and refer to a sequence, such as an amino acid sequence or a nucleic acid sequence that includes a deletion, insertion, replacement or interruption of a naturally occurring sequence. Often in the context of the invention, a substituted sequence shall refer, for example, to the replacement of a naturally occurring residue.

As used herein, "modified enzyme" refers to an enzyme that includes a deletion, insertion, replacement or interruption of a naturally occurring sequence.

The term "variant" refers to a region of a protein that contains one or more different amino acids as compared to a reference protein, for example, a naturally occurring or wild-type protein.

As used herein the term "parental strain" refers to the strain of fungal cells that existed prior to exposure to an agent that generates genetic diversity. In some embodiments, the parental strain is already a highly productive strain or possesses favorable or desired characteristics. After exposure to the agent, the "parental cells" becomes a population of genetically diverse test cells. Due to the random nature of the process for generating genetic diversity, many different types of cells are expected to be generated. Therefore, when the selection techniques are applied, a large number of different test cells are placed under selection.

The term "test cells" or "population of testing filamentous fungi" as used interchangeably herein refers, in general, to genetically diverse fungal cells generated from a parent strain of filamentous fungus. The term also encompasses any progeny of the test cell.

"Improved cells" are isolated for their growth characteristics, level of enzyme production and/or other selection criteria as determined in the assays described herein. The term also encompasses any progeny of the improved cell. The improved cell(s) can be isolated and propagated to establish a new improved fungal strain. Non-limiting examples of improved cells exhibit the following characteristics: (i) an increased efficiency of carbon usage compared to the parental cells; (ii) an improved specific activity of one or more proteins or enzymes produced compared to the parental cells; (iii) an overall improved yield determined, for example, by measuring gram of protein or enzyme produced per gram of carbon input; (iv) a higher production level of one or more proteins or enzymes at normal temperature; and/or (v) the ability to maintain a level of protein or enzyme production that is better than the parental cells at elevated temperatures.

As a non-limiting example, the normal temperature range for the production of cellulases by *Trichoderma-reesei* is 24°C to 28°C. According to the present teachings, the selection temperature for *Trichoderma reesei* can be 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, or 48°C. In some embodiments, the culturing is performed in liquid phase. Depending on the experimental design, a range of selection temperatures can be used. Choice of selection temperatures for the culturing depends on other factors, such as but not limited to growth rate, viability, and the distribution of different types of cellulases that is produced by the test cells and will be determinable by one of skill in the art.

The terms "cellulase" or "cellulolytic enzymes" are used interchangeably and refer to a category of enzymes capable of hydrolyzing cellulose (beta-1,4-glucan or beta D-glucosidic linkages) polymers to shorter cello-oligosaccharide oligomers, cellobiose and/or glucose. In general, the category of enzymes include, but are not limited to: (i) endoglucanases (EG) or 1,4-β-d-glucan-4-glucanohydrolases (EC 3.2.1.4), (ii) exoglucanases, including 1,4-β-d-glucan glucanohydrolases (also known as cellodextrinases) (EC 3.2.1.74) and 1,4-β-d-glucan cellobiohydrolases (exo-cellobiohydrolases, CBH) (EC 3.2.1.91), and (iii) β-glucosidases (BG) or β-glucoside glucohydrolases (EC 3.2.1.21).

The term "exo-cellobiohydrolase" (CBH) refers to a group of cellulase enzymes classified as EC 3.2.1.91 and/or those in certain GH families, including, but not limited to, those in GH families 5, 6, 7, 9 or 48. These enzymes are also known as exoglucanases or cellobiohydrolases. CBH enzymes hydrolyze cellobiose from the reducing or non-reducing end of cellulose. In general a CBHI type enzyme preferentially hydrolyzes cellobiose from the reducing end of cellulose and a CBHII type enzyme preferentially hydrolyzes the non-reducing end of cellulose.

The term "cellobiohydrolase activity" is defined herein as a 1,4-D-glucan cellobiohydrolase activity which catalyzes the hydrolysis of 1,4-beta-D-glucosidic linkages in cellulose, cellotetriose, or any beta-1,4-linked glucose containing polymer, releasing cellobiose from the ends of the chain. As used herein, cellobiohydrolase activity is determined by release of water-soluble reducing sugar from cellulose as measured by the PHBAH method of Lever et al., 1972, Anal. Biochem. 47: 273-279. A distinction between the exoglucanase mode of attack of a cellobiohydrolase and the endoglucanase mode of attack is made by a similar measurement of reducing sugar release from substituted cellulose such as carboxymethyl cellulose or hydroxyethyl cellulose (Ghose, 1987, Pure & Appl. Chem. 59: 257-268). A true cellobiohydrolase will have a very high ratio of activity on unsubstituted versus substituted cellulose (Bailey et al, 1993, Biotechnol. Appl. Biochem. 17: 65-76).

The term "endoglucanase" (EG) refers to a group of cellulase enzymes classified as EC 3.2.1.4, and/or those in certain GH families, including, but not limited to, those in GH families 5, 6, 7, 8, 9, 12, 17, 31, 44, 45, 48, 51, 61, 64, 74 or 81. An EG enzyme hydrolyzes internal beta-1,4 glucosidic bonds of the cellulose. The term "endoglucanase" is defined herein as an endo-1,4-(1,3;1,4)-beta-D-glucan 4-glucanohydrolase which catalyses endohydrolysis of 1,4-beta-D-glycosidic linkages in cellulose, cellulose derivatives (for example, carboxy methyl cellulose), lichenin, beta-1,4 bonds in mixed beta-1,3 glucans such as cereal beta-D-glucans or xyloglucans, and other plant material containing cellulosic components. As used herein, endoglucanase activity is determined using carboxymethyl cellulose (CMC) hydrolysis according to the procedure of Ghose, 1987, Pure and Appl. Chem. 59: 257-268.

The term "beta-glucosidase" is defined herein as a beta-D-glucoside glucohydrolase classified as EC 3.2.1.21, and/or those in certain GH families, including, but not limited to, those in GH families 1, 3, 9 or 48, which catalyzes the hydrolysis of cellobiose with the release of beta-D-glucose. As used herein, beta-glucosidase activity may be measured by methods known in the art, e.g., HPLC. "Cellulolytic activity" encompasses exoglucanase activity, endoglucanase activity or both types of enzyme activity, as well as beta-glucosidase activity.

The term "filamentous fungi" means any and all filamentous fungi recognized by those of skill in the art. In general, filamentous fungi are eukaryotic microorganisms and include all filamentous forms of the subdivision Eumycotina. These fungi are characterized by a vegetative mycelium with a cell wall composed of chitin, beta-glucan, and other complex polysaccharides. In some embodiments, the filamentous fungi of the present teachings are morphologically, physiologically, and genetically distinct from yeasts. In some embodiments, the filamentous fungi include, but are not limited to the following genera: *Aspergillus, Acremonium, Aureobasidium, Beauveria, Cephalosporium, Ceriporiopsis, Chaetomium paecilomyces, Chrysosporium, Claviceps, Cochiobolus, Cryptococcus, Cyathus, Endothia, Endothia mucor, Fusarium, Gilocladium, Humicola, Magnaporthe, Myceliophthora, Myrothecium, Mucor, Neurospora, Phanerochaete, Podospora, Paecilomyces, Penicillium, Pyricularia, Rhizomucor, Rhizopus, Schizophylum, Stagonospora, Talaromyces, Trichoderma, Thermomyces, Thermoascus, Thielavia, Tolypocladium, Trichophyton,* and *Trametes pleurotus*. In some embodiments, the filamentous fungi include, but are not limited to the following: *A. nidulans, A. niger, A. awomari, e.g.*, NRRL 3112, ATCC 22342 (NRRL 3112), ATCC 44733, ATCC 14331 and strain UVK 143f, *A. oryzae, e.g.*, ATCC 11490, *N. crassa, Trichoderma reesei, e.g.*, NRRL 15709, ATCC 13631, 56764, 56765, 56466, 56767, and *Trichoderma viride, e.g.*, ATCC 32098 and 32086.

The term "*Trichoderma*" or *"Trichoderma* species" used herein refers to any fungal organisms which have previously been classified as a *Trichoderma* species or strain, or which are currently classified as a *Trichoderma* species or strain, or as a *Hypocrea* species or strain. In some embodiments, the species include *Trichoderma longibrachiatum, Trichoderma reesei, Trichoderma viride,* or *Hypocrea jecorina*. Also contemplated for use as an original strain are cellulase-overproducing strains such as *T. longibrachiatum*/*reesei* RL-P37 (Sheir-Neiss et al., Appl. Microbiol. Biotechnology, 20 (1984) pp. 46-53; Montenecourt B.S., Can., 1-20, 1987), and RUT-C30 (ATCC No. 56765) and strain QM9414 (ATCC No. 26921).

In some embodiments, the present teachings provide a method for screening for a desired filamentous fungus by screening a population of testing filamentous fungi for a subpopulation of filamentous fungi with a predetermined rate of metabolism and selecting a desired filamentous fungus from the subpopulation of filamentous fungi based on a pre-determined selection parameter. The population of testing filamentous fungi can be suitable population from which a desired filamentous fungus can be selected. In some embodiments, the population of testing filamentous fungi is a population of genetically diverse filamentous fungi. Methods of obtaining a genetically diverse population of testing filamentous fungi are known to one of skill in the art and exemplary methods are described herein. In some embodiments, the population of testing filamentous fungi is a population of filamentous fungi after exposure to a mutation inducing condition.

According to the present teachings, a population of genetically diverse filamentous fungi can be obtained by exposing the population of filamentous fungi to an agent that generates genetic diversity in the genome of the cells. In one embodiment, the agent that generates genetic diversity is a mutagen that causes localized nucleotide change(s) in the genome. Parental cells and test cells may be mutagenized by such mutagens using any methods known in the art. For example, mutagenesis of the cells can be achieved by irradiation, e.g., ultraviolet light, X-ray, or gamma radiation. Alternatively, mutagenesis can be achieved by treatment with chemical mutagens, *e.g.*, nitrous acid, nitrosamines, methyl nitrosoguanidine, ethylmethanesulfonate, and base analogues such as 5-bromouracil. In one embodiment, insertional mutagenesis is used using transposons, restriction enzyme-mediated integration ("REMI") or Agrobacterium-mediated transformation.

In some embodiments, the agent that generates genetic diversity in the parental cells or test cells is a cytogenetic agent that causes gross changes in the genome, generally at the cytogenetic or chromosomal level, such as but not limited to autopolyploid formation, micronuclei formation, polykaryon formation, chromosomal rearrangement, chromosomal reassortment, chromosomal aberration, chromatid loss, large-scale recombination, etc. Many such agents are known, including but not limited to colchicine (commonly used at 0.1% w/v).

In some embodiments, the mutagen is applied to spores of the parental strain or test strain, and the surviving spores are plated out on a solid medium. The cells are plated out at various cell densities to facilitate growth and identification by visual inspection or other means. In other embodiments, other forms of the fungal organism beside spores can also be used in the genetic diversification step. In some embodiments, the agent is a mutagen that is applied at a dose that produces a lethality of about 1-99.9 %. In various embodiments, the agent is a mutagen that is applied at a dose that produces a lethality of about 50%, about 60%, about 70%, about 80%, about 90%, or about 95%. In some embodiments, the mutagen is nitrosoguanidine (N-methyl-N'-nitro-N-nitrosoguanidine - MNNG) or ethyl methane sulfonate (nitrogen mustard gas). (*see*, Gerhardt et al. 1994, Methods for general and molecular bacteriology, American Society for microbiology, p. 297-316).

The population of testing filamentous fungi can be screened for a subpopulation of filamentous fungi with a predetermined rate of metabolism by any means now known, or later discovered, in the art. In some embodiments, the genetically diverse population of testing filamentous fungi are cultured in a medium comprising a substrate metabolized by the filamentous fungi and the subpopulation of filamentous fungi with a predetermined rate of metabolism are those fungi that exhibit growth rates higher than those of the rest of the population of filamentous fungi. The substrate provided for determining the rate of metabolism can be any substrate known to one of skill in the art that allows for discernable differences in the rate of metabolism. Any minimal medium known in the art for culturing filamentous fungi can be used to prepare the medium. In some embodiments, the substrate is resistant to microbial degradation. In some embodiments, the substrate is cellulose. In some embodiments, the medium comprises cellulose and cellulose is the sole source of carbon and energy. In some embodiments, the medium is substantially free of disaccharides and monosaccharides. In some embodiments, the culturing is performed in a solid phase that enables the culturing and screening of a large population of test cells in batches. In some embodiments, the cellulose is purified cellulose which includes but is not limited to microcrystalline cellulose, such as AVICEL^{®} (FMC Biopolymer, Philadelphia, PA) that, in water, with shear, forms a three-dimensional matrix comprised of insoluble microcrystals that form an extremely stable, thixotropic gel.

In some embodiments, the screening of the population of testing filamentous fungi for the subpopulation can be carried out in a solid phase assay. A suitable, non-limiting example of a solid phase assay is the Cellulose Plate Screen (CPS). The population of testing filamentous fungi are cultured in two layers of solid medium, each layer of medium comprising cellulose as the sole source or a limiting source of energy or carbon. The two layers may comprise the same ingredients and even the same concentrations of ingredients, and are preferably prepared in a plate. The solid media preferably comprises agar, e.g., 1.5% (w/v) agar. The bottom layer comprises a population of test cells, preferably spores, while the top layer does not comprise any fungal cells. The plate comprising a top layer and the fungal cells in the bottom layer are incubated at a temperature for a period of time for the fungal cells to grow within the solid medium. The thickness of the top layer is uniform across the plate and controlled so that the fastest growing fungal cells that consume cellulose emerge at the surface of the top layer. In some embodiments, the improved cells of the subpopulation of filamentous fungi are those that exhibit growth rates higher than that of the 50^{th}, 60^{th}, 70^{th}, 80^{th}, 90^{th}, 95^{th}, or 98^{th} percentile in growth rate of said population of test cells. The test cells that break the surface of the top layer are visually detectable and can be readily isolated by techniques known in the art. Typically, the thickness of the top layer ranges from about 2.5mm, 5mm, 7.5mm, 10mm, 12.5mm, 15mm, 17.5mm, 20mm, 25mm to about 30mm.

In some embodiments, this solid phase assay can be used independently or at the end of the selection regime to facilitate isolation of the improved cells and ranking the improved cells by growth rate in cellulose. In some embodiments, the solid phase assay can also be carried out at a temperature that is higher than the temperature at which the population of testing filamentous fungi normally produce a cellulase enzyme.

The present teachings further provide for the selection of the desired filamentous fungus from the subpopulation of filamentous fungi based on a pre-determined selection parameter. The pre-determined selection parameter may be any assayable parameter and indicates, for example, the efficiency of carbon usage by a testing filamentous fungus. The carbon usage efficiency can be assayed by a variety of parameters, including, but not limited to, the yield of one or more desired polypeptides or enzymes produced by the filamentous fungus, the productivity of the filamentous fungus, and the specific activity of the enzymes produced by the filamentous fungus. In some embodiments, the pre-determined selection parameter indicates thermal stability determined, for example, by a higher production level of one or more protein or enzymes at normal temperature or by the ability to maintain a level of protein or enzyme production that is better than the parental cells at elevated temperatures.

According to the present teachings, the yield can be determined, for example, by measuring quantity (gram) of polypeptide or enzyme produced per gram of carbon input. In some embodiments, the input carbon corresponds to the amount of carbon provided to the filamentous fungus, or, it could correspond to the amount of carbon consumed by the testing filamentous fungus. The productivity can be determined, for example, by measuring production of a protein, e.g., quantity (gram) of polypeptide or enzyme produced in a pre-determined period of time relative to a pre-determined amount of testing filamentous fungus. In some embodiments, the methods of the present teachings provide for the selection of filamentous fungus that produce a polypeptide or an enzyme with increased yield, increased productivity, increased specific activity, or any combination thereof. In some embodiments, the filamentous fungus further produces a polypeptide or enzyme that has improved thermal stability.

The desired enzymes or polypeptides of the present teachings include, but are not limited to, cellulase, xylanase, glucoamylase, amylase, laccase, protease, phytase or hemicellulase. In some embodiments, the selection parameter indicates the carbon usage efficiency of a filamentous fungus producing a cellulase, xylanase, glucoamylase, amylase, laccase, protease, phytase or hemicellulase. In some embodiments, the selection parameter further comprises an indicator corresponding to productivity of a cellulase, xylanase, glucoamylase, amylase, laccase, protease, phytase or hemicellulase expressed in the testing filamentous fungus.

In some embodiments, the population of testing filamentous fungi are pre-selected filamentous fungi. The pre-selection criteria can be any assayable criteria, including, but not limited to, resistance to an antibiotic agent. The antibiotic can be any antibiotic known in the art and includes, but is not limited to Bialaphos, cyclohexamidine, Tunicamycin, Griseofulvin, Nikkomycin Z, caspofungin, Actinomycin D, Brefeldin A, and hygromycin and polyene antibiotics. Suitable non-limiting examples polyene antibiotics include nystatin and Amphotericin B.

The methods of the present teachings can be used for screening for filamentous fungi of any desirable strain. In some embodiments, the testing filamentous fungus is selected from the group consisting of *Aspergillus, Acremonium, Aureobasidium, Beauveria, Cephalosporium, Ceriporiopsis, Chaetomium, Paecilomyces, Chrysosporium, Claviceps, Cochiobolus, Cryptococcus, Cyathus, Endothia, Fusarium, Gilocladium, Humicola, Magnaporthe, Myceliophthora, Myrothecium, Mucor, Neurospora, Phanerochaete, Podospora, Paecilomyces, Penicillium, Pyricularia, Rhizomucor, Rhizopus, Schizophylum, Stagonospora, Talaromyces, Trichoderma, Thermomyces, Thermoascus, Thielavia, Tolypocladium, Trichophyton, Trametes,* and *Pleurotus*. In some embodiments, the testing filamentous fungus is Trichoderma. In some embodiments, the testing filamentous fungus is *T. reesei*. In some embodiments, the testing filamentous fungus is *T. reesei* that uses cellulose as its carbon source.

In some embodiments, the present teachings provide a filamentous fungus obtained by the methods discussed above. In some embodiments, the filamentous fungus is *Trichoderma, e.g.*, *T. reesei*. In some embodiments, the filamentous fungus obtained by the methods of the present teachings is T. *reesei* and it produces a mixture of cellulases with a predetermined activity. The activity can be any activity for which a strain with improved properties is desired. In some embodiments, the activity is saccharification.

In some embodiments, the present teachings provide a method for screening for a desired filamentous fungus by screening a population of testing filamentous fungi based on a combination of two or more selection parameters. In some embodiments, the population of testing filamentous fungi is screened based on two selection parameters, a first selection parameter and a second selection parameter. Any combination of selection parameters can be chosen to obtain the desired improved strain of filamentous fungus. In some embodiments, the first selection parameter indicates the rate of metabolism of a testing filamentous fungus and the second selection parameter indicates the ability of a testing filamentous fungus to resist an antibiotic. In some embodiments, the first selection parameter indicates the rate of metabolizing a substrate that is resistant to microbial degradation, *e.g.*, cellulose.

In some embodiments, the present teachings provide a method for screening for a desired filamentous fungus by screening a population of testing filamentous fungi based on a pre-determined selection parameter that indicates the rate of metabolism of a testing filamentous fungus of, for example, a substrate that is resistant to microbial degradation. Here the population of testing filamentous fungi has been pre-selected against an antibiotic.

Aspects of the present teachings may be further understood in light of the following examples, which should not be construed as limiting the scope of the present teachings. It will be apparent to those skilled in the art that many modifications, both to materials and methods, may be practiced without departing from the present teachings.

### 7. EXAMPLES

### EXAMPLE 7.1 Mutant Preparation, Media and Solutions

Cellulase Screening Medium was used to isolate variants with improved protein production. Cellulase Screening Medium contained 20ml of 50X Vogels stock solution, 0.5g of AVICEL^{®} (FMC Biopolymer, Philadelphia, PA), and 20g of Agar, 980ml of dH₂O. 50X Vogels Stock solution was prepared by dissolving: (1) 150 g of Na₃Citrate•2H₂O; 10 g of MgSO₄•7H₂O; and 5 g of CaCl₂•2H₂O in 300 ml of dH₂O; (2) 250 g of KH₂PO₄ in 500 dH₂O; (3) 100 g of NH₄NO₃ in 200 ml of dH₂O. The solutions were added together and 5 ml of Vogels Trace Element Solution and 2.5 ml of Vogels Biotin Solution was added. Vogels trace elements solution contained 1 liter of dH₂O, 50g of Citric Acid; 50g of ZnSO₄•7H₂O; 10g of Fe(NH₄)2SO₄•6H₂O; 2.5g of CuSO₄•5H₂O; 0.5g of MnSO₄•4H₂O; 0.5g of H₃BO₃ (Boric Acid); and 0.5g of NaMoO₄ 2H₂O. (*see* Davis et al., 1970, Methods in Enzymology 17A, 79 -143; and Davis et al., 2000, Neurospora, Contributions of a Model Organism, Oxford University Press, for information on Vogels minimal medium). Vogels biotin solution comprises 0.1g of d-Biotin in 1 liter of water. Total protein production in shake flasks was examined by incubating mutants at 28 °C, 150 rpm , for 96h in 250 ml flasks containing 50 mL Lactose Minimal Medium as described by Ilmen et al., 1997, App Environ Microbiol 63, 1298-1306 except that 100mM piperazine-N,N'-bis(2-ethanesulfonic acid) (PIPES; Calbiochem) was included to maintain the pH at 5.5. Protein assays were done on supernatants after TCA precipitation using a BCA assay provided by Pierce (Rockford, IL).

Any minimal medium known in the art for culturing filamentous fungi can be used to prepare the medium for use in the culturing step.

The following experiments began with the *Trichoderma reesei* strain A83 which is a highly productive strain related to the strain RL-P37. To generate genetic diversity, the cells were mutated with the methylating compound N-methyl-N'-nitro-N-nitrosoguanidine (NTG). NTG is one of the most potent mutagens available; it induces primarily base transition mutations of the GC to AT type (although AT to GC transitions, transversions, and frameshifts arise at low frequencies). A kill curve was prepared when a strain was mutated for the first time. Starting at time zero, samples were taken every 30 minutes and a viable spore count was conducted. Once the kill curve was established, only the time zero and the final viable count were made to ensure the correct % kill had been obtained. For example, a 50% kill library and a 99% kill library were prepared by incubating the spores with NTG for 1.5 hours, and 3 hours, respectively. Improved strains derived from RL-P37 including A83, T4, NY27, and 41G obtained using a 99% kill library. After incubation, the NTG was removed by washing the spores at least three times in water. Aliquots were prepared of the mutated spores and they were stored in glycerol at -70°C.

Fresh fungal plates were prepared and used to obtain a spore suspension containing about 1 x 109 spore forming units /ml. The number of spore forming units /ml was determined using a hemocytometer. A solution of NTG (Aldrich-4991) was freshly prepared to a concentration of 15 mg/mL in DMSO and added at a final concentration of 1.0mg/ml to the fungal spore suspension. The fungal spore suspension was then incubated at room temperature in the dark until the desired kill of 99% was obtained.

Isolation of T4. Cellulase screening medium was prepared and cooled to 55°C in a water bath. In a small petri dish (82mm), an aliquot containing about one million mutated spores was dispensed in a circle about ½ way between the center of the plate and the edge and 10 ml of the Cellulase Screening Medium (described above) was added. The plate was subsequently swirled so that the spores were dispersed in the middle of the plate, but not dispersed all the way to the edges, and set to harden for about 5-10 minutes. 25 ml of Cellulase Screening medium was added and allowed to harden. Another 10 ml of Cellulase Screening medium was then added and the plates were incubated at 28°C overnight. The next day, the surface of the plate was checked every four hours for growth using a dissecting microscope. For each library, the approximate time that colonies reached the surface of the plate was determined.

The first 1-3 isolates that reached the surface of the agar were collected using a sterile razor blade, ignoring the colonies that came up around the edges. The colony was observed under the microscope and the razor blade was used for touching the surface of the colony, being careful not to dig into the agar. A small piece of mycelia was removed and placed onto PDA and incubated 28 °C. Once grown, the isolates were evaluated for total protein production in shake flasks.

After the first round of screening, T4 was found to produce about 25% more total protein compared to parent A-83 (Table 1).

Table 1. Protein production in shake flasks by RL P-37, A-83, T4, NY27, and 41 G.

| Strain # | Protein g/L |
|---|---|
| RL P-37 | 0.6 +/- 0.04 |
| A-83 | 0.9 +/- 0.07 |
| T4 | 1.2 +/- 0.07 |
| NY27 | 1.8 +/- 0.05 |
| 41G | 2.4 +/- 0.10 |

Isolation of NY27. T4 spores were mutated with NTG until a 99% kill was obtained as described above. The mutated spores were plated out on to PDA agar (Difco) containing 10 µg/mL nystatin.

Polyene antibiotics such as nystatin, bind to the sterol, ergosterol, in the fungal cell membrane causing the cytoplasmic membrane to leak resulting in death. While not bound to any theory, improved protein production may be due to improved secretion resulting from alterations in the cell membrane resulting in increased permeability and/or overexpression of efflux transporter genes

Colonies resistant to Nystatin were isolated and examined for total protein production in shake flasks. One of the best mutants was NY 27 producing about 33% more total protein than T4 in shake flasks (Table 1).

Isolation of 41 G. NY27 was mutated with NTG until a kill of 99% was obtained. The mutated spores were screen in a identical manner as in the isolation of T4.

Cellulase screening medium was prepared and cooled to 55°C in a water bath. In a small petri dish (82mm), an aliquot containing about one million mutated spores was dispensed in a circle about ½ way between the center of the plate and the edge and 10 ml of the Cellulase Screening Medium (described above) was added. The plate was subsequently swirled so that the spores were dispersed in the middle of the plate, but not dispersed all the way to the edges, and set to harden for about 5-10 minutes. 25 ml of Cellulase Screening medium was added and allowed to harden. Another 10 ml of Cellulase Screening medium was then added and the plates were incubated at 28°C overnight. The next day, the surface of the plate was checked every four hours for growth using a dissecting microscope. For each library, the approximate time that colonies reached the surface of the plate was determined.

The first 1-3 isolates that reached the surface of the agar were collected using a sterile razor blade, ignoring the colonies that came up around the edges. The colony was observed under the microscope and the razor blade was used for touching the surface of the colony, being careful not to dig into the agar. A small piece of mycelia was removed and placed onto PDA and incubated 28 °C. Once grown, the isolates were evaluated for total protein production in shake flasks.

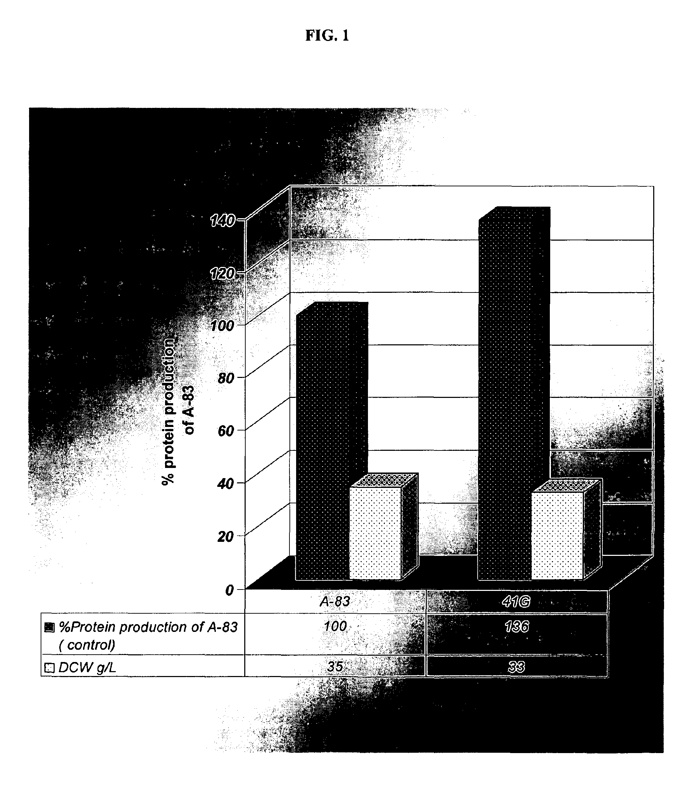

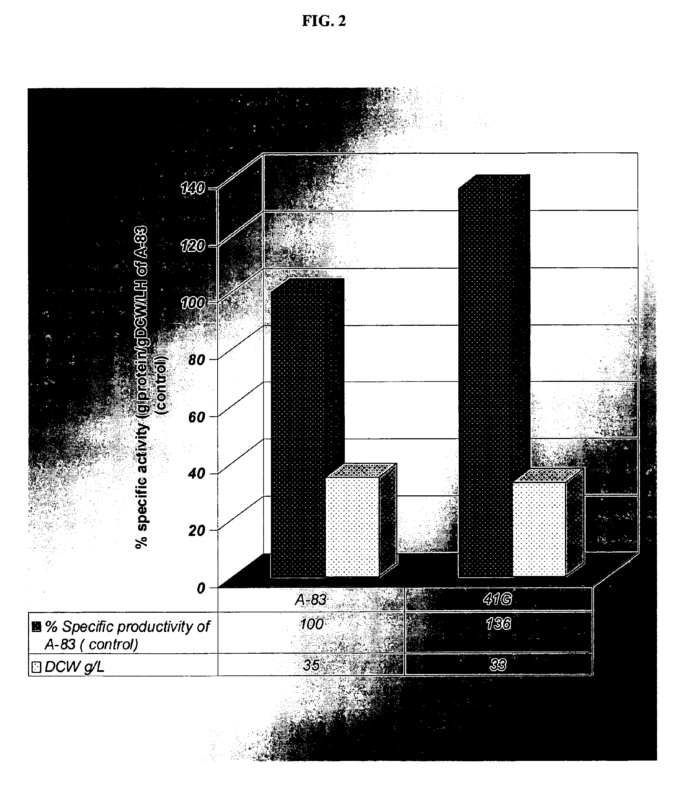

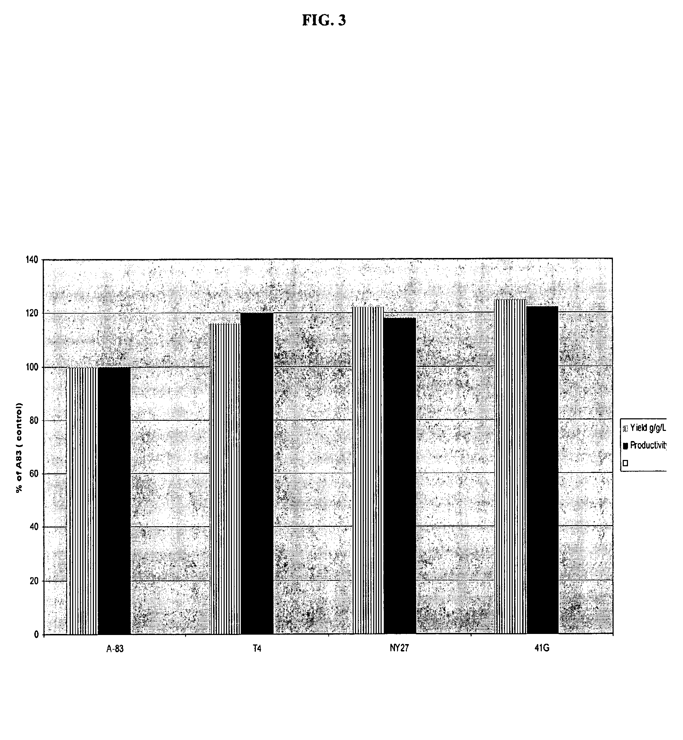

Mutant 41G was found to produce about 25% more total protein compared to parent NY27 (Table 1). Figure 1 shows production of total protein by A83 and 41 G in 120 h in a 14L cellulase fermentation. Total protein is shown as % of A83 (control). Dry cell weight (DCW) is in g/L. DCW is measured by filtering a known amount of whole broth to remove the cell mass, drying the cell mass, and determining the weight (g) per liter of whole broth. Figure 2 shows the specific productivity of 41 G in 120 h in 14L cellulase fermentation compared to A-83 control. Specific productivity (grams of protein per gram of DCW/ LH) is shown as % of A-83 ( control). Dry cell weight (DCW) is in g/L. Figure 3 shows the evolution of 41G using the Cellulose Plate Screen (CPS) and nystatin resistance screen and improvements in yield (grams of protein/grams of carbon/LH) and productivity (grams of protein/LH) in 14L fermentors strains in 41 G the 41 G lineage. Results are shown as % of A-83 (control).

Mutant T4 and 41 G were grown in 14L fermentors in fed batch fermentations optimal for cellulase production using medium as described by Ilmen et al., 1997, App Environ Microbiol 63, 1298-1306 except that 100mM piperazine-N,N'-bis(2-ethanesulfonic acid) (PIPES; Calbiochem). Other methods are typical for those skilled in the art. Figure 3 shows productivity and yield improvements relative to strain A-83.

### EXAMPLE 7.2: Saccharification

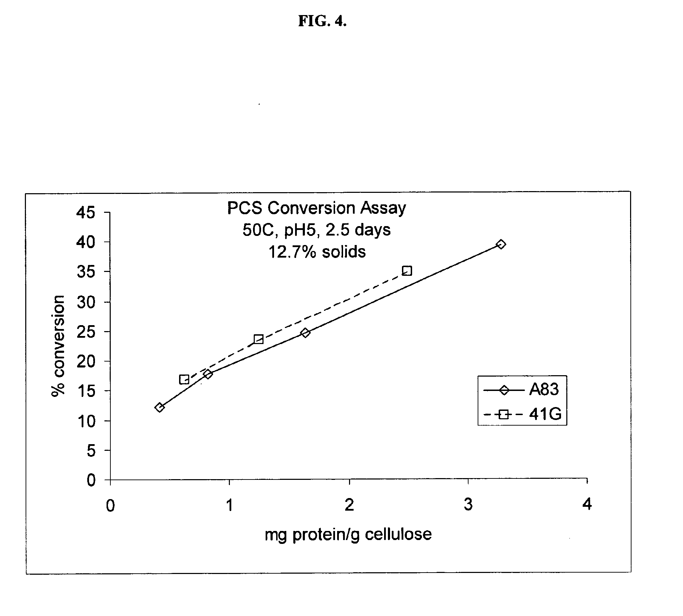

T4 reactions were carried out in a standard microtiter plate assay at 50 °C for 5 days with acid pretreated corn stover (13% solids, approximately 7% cellulose) with 5, 10, and 20 milligrams cellulase per gram cellulose. There were seventeen different A83 fermentation samples (run in duplicate) averaged and four different T4 samples (run in duplicate and quadruplicate) averaged. T4 cellulase out performed A-83 cellulase in the plate assay. For 41G, 8 mg/g cellulose containing 4.3 % (w/v) solids of PYP (dilute -acid-pretreated yellow popular which contained 2.5% (w/v) cellulose was dosed with equal amounts of A-83 and 41 G and incubated at 38 C , pH 5.0 with continuous mixing by inversion (every 20 min) in sealed tubes for 168 h. The % saccharification of PYP cellulose was determined overtime. Using cellulase produced by T4, the % saccharification was improved by 10-15% compared to A-83 cellulase. The 4 1 G cellulase showed improved % saccharification compared to A-83 cellulase (Figure 4).

The following numbered paragraphs contain statements of broad combinations of the inventive technical features herein disclosed:-
1. A method for screening for a desired filamentous fungus comprising screening a population of testing filamentous fungi for a subpopulation of filamentous fungi with a predetermined rate of metabolism and selecting a desired filamentous fungus from the subpopulation of filamentous fungi based on a pre-determined selection parameter, wherein the selection parameter indicates carbon usage efficiency of a testing filamentous fungus.
2. The method of paragraph 1, wherein the subpopulation of filamentous fungi is obtained by selecting filamentous fungi based on metabolizing rate of a substrate, wherein the substrate is resistant to microbial degradation.
3. The method of paragraph 1, wherein the selection parameter corresponds to production of a protein in the testing filamentous fungus relative to a predetermined amount of carbon provided to the testing filamentous fungus.
4. The method of paragraph 1, wherein the selection parameter corresponds to production of a cellulase, xylanase, glucoamylase, amylase, laccase, protease, phytase or hemicellulase expressed by the testing filamentous fungus relative to a predetermined amount of carbon provided to the testing filamentous fungus.
5. The method of paragraph 1, wherein the selection parameter corresponds to production of a protein in the testing filamentous fungus relative to a predetermined amount of carbon consumed by the testing filamentous fungus.
6. The method of paragraph 1, wherein the selection parameter further comprises an indicator corresponding to the productivity of a protein in the testing filamentous fungus.
7. The method of paragraph 1, wherein the productivity of the protein in the testing filamentous fungus is determined by production of a protein in the testing filamentous fungus in a predetermined period of time relative to a predetermined amount of the testing filamentous fungus.
8. The method of paragraph 1, wherein the selection parameter further comprises an indicator corresponding to production of a cellulase, xylanase, glucoamylase, amylase, laccase, protease, phytase or hemicellulase expressed in the testing filamentous fungus relative to a predetermined amount of the testing filamentous fungus.
9. The method of paragraph 1, wherein the screening the population of testing filamentous fungi for the subpopulation is carried out in a solid phase assay.
10. The method of paragraph 1, wherein the population of testing filamentous fungi is a population of filamentous fungi pre-selected against an antibiotic.
11. The method of paragraph 1, wherein the population of testing filamentous fungi is a population of filamentous fungi pre-selected against a polyene antibiotic.
12. The method of paragraph 10, wherein the antibiotic is selected from the group consisting of nystatin, Amphotericin B, bialaphos, cyclohexamidine, Tunicamycin, Griseofulvin, Nikkomycin Z, caspofungin, Actinomycin D, Brefeldin A, and hygromycin.
13. The method of paragraph 1, wherein the testing filamentous fungus is selected from the group consisting of Aspergillus, Acremonium, Aureobasidium, Beauveria, Cephalosporium, Ceriporiopsis, Chaetomium, Paecilomyces, Chrysosporium, Claviceps, Cochiobolus, Cryptococcus, Cyathus, Endothia, Fusarium, Gilocladium, Humicola, Magnaporthe, Myceliophthora, Myrothecium, Mucor, Neurospora, Phanerochaete, Podospora, Paecilomyces, Penicillium, Pyricularia, Rhizomucor, Rhizopus, Schizophylum, Stagonospora, Talaromyces, Trichoderma, Thermomyces, Thermoascus, Thielavia, Tolypocladium, Trichophyton, Trametes, and Pleurotus.
14. The method of paragraph 1, wherein the testing filamentous fungus is T. reesei and it uses cellulose as carbon source.
15. The method of paragraph 1, wherein the testing filamentous fungus uses cellulose as carbon source.
16. A filamentous fungus obtained by the method of paragraph 1.
17. A filamentous fungus obtained by the method of paragraph 1, wherein the filamentous fungus is T. reesei and it produces a mixture of cellulases with a predetermined activity.
18. The filamentous fungus of paragraph 17, wherein the activity is saccharification.
19. A method for screening for a desired filamentous fungus comprising screening a population of testing filamentous fungi based on a first selection parameter and a second selection parameter, wherein the first selection parameter indicates the rate of metabolism of a testing filamentous fungus and the second selection parameter indicates the ability of a testing filamentous fungus to resist an antibiotic.
20. The method of paragraph 19, wherein the first selection parameter indicates the rate of metabolizing a substrate that is resistant to microbial degradation.
21. A method for screening for a desired filamentous fungus comprising screening a population of testing filamentous fungi based on a pre-determined selection parameter, wherein the pre-determined selection parameter indicates the rate of metabolism of a testing filamentous fungus and wherein the population of testing filamentous fungi is a population of filamentous fungi pre-selected against an antibiotic.
22. The method of paragraph 21, wherein the pre-determined selection parameter indicates the rate of metabolizing a substrate that is resistant to microbial degradation.

## Claims

1. A method for screening for a filamentous fungus comprising:
i) screening a population of testing filamentous fungi against a substrate for a subpopulation of filamentous fungi with a predetermined rate of metabolism that exhibit growth rates higher than that of the 50^{th} percentile in growth rate of said population of test cells and
ii) screening the population of testing filamentous fungi against an antibiotic for the subpopulation of filamentous fungi having resistance to the antibiotic,
iii) selecting a filamentous fungus from the subpopulation of filamentous fungi based on at least two pre-determined selection parameters, wherein the selection parameters comprise:
a.) the rate of metabolizing the substrate by the subpopulation of filamentous fungus, wherein the substrate is resistant to microbial degradation and
b.) the ability of the subpopulation of filamentous fungus to resist the antibiotic.

2. The method of claim 1, wherein the selected filamentous fungi is obtained by selecting filamentous fungi based on metabolizing rate of a substrate, wherein the substrate is resistant to microbial degradation.

3. The method any one of claims 1-2, wherein the substrate is cellulose.

4. The method of claim 3, wherein the cellulose is the sole source of carbon and energy.

5. The method of any one of claims 1-4, wherein the selection parameter further comprises measuring an increase in production of a protein in the selected filamentous fungus relative to a predetermined amount in the testing filamentous fungus.

6. The method of any one of claims 1-5, wherein the selection parameter further comprises an indicator corresponding to the productivity of a protein in the testing filamentous fungus.

7. The method of any one of claims 1-6, wherein the selection parameter further comprises measuring production of a cellulase, xylanase, glucoamylase, amylase, laccase, protease, phytase or hemicellulase expressed by the selected filamentous fungus relative to a predetermined amount of the testing filamentous fungus.

8. The method of any one of claims 1-7, wherein the productivity of the protein in the testing filamentous fungus is determined by production of a protein in the selected filamentous fungus in a predetermined period of time relative to a predetermined amount of the testing filamentous fungus.

9. The method of any one of claims 1-8, wherein the selection parameter further comprises an indicator corresponding to production of a cellulase, xylanase, glucoamylase, amylase, laccase, protease, phytase or hemicellulase expressed in the selected filamentous fungus relative to a predetermined amount of the testing filamentous fungus.

10. The method of any one of claims 1-9, wherein the screening the population of testing filamentous fungi for the selected filamentous fungus is carried out in a solid phase assay.

11. The method of any one of claims 1-10, wherein the subpopulation of testing filamentous fungi is a population of selected filamentous fungi resistant to a polyene antibiotic.

12. The method of claim 11, wherein the antibiotic is selected from the group consisting of nystatin, Amphotericin B, bialaphos, cyclohexamidine, Tunicamycin, Griseofulvin, Nikkomycin Z, caspofungin, Actinomycin D, Brefeldin A, and hygromycin.

13. The method of any one of claims 1-12, wherein the testing filamentous fungus is selected from the group consisting of *Aspergillus, Acremonium, Aureobasidium, Beauveria, Cephalosporium, Ceriporiopsis, Chaetomium, Paecilomyces, Chrysosporium, Claviceps, Cochiobolus, Cryptococcus, Cyathus, Endothia, Fusarium, Gilocladium, Humicola, Magnaporthe, Myceliophthora, Myrothecium, Mucor, Neurospora, Phanerochaete, Podospora, Paecilomyces, Penicillium, Pyricularia, Rhizomucor, Rhizopus, Schizophylum, Stagonospora, Talaromyces, Trichoderma, Thermomyces, Thermoascus, Thielavia, Tolypocladium, Trichophyton, Trametes,* and *Pleurotus*.

14. The method of claim 1, wherein the selected filamentous fungus is T. *reesei.*

15. A filamentous fungus obtained by the method of claim 1, wherein the selected filamentous fungus is optionally *T. reesei* and it produces a mixture of cellulases with a predetermined activity,
wherein, optionally, the activity is saccharification.
